# EUROPÄISCHE PATENTSCHRIFT

(11) **EP 2 192 857 B1**
(45) Veröffentlichungstag und Bekanntmachung des Hinweises auf die Patenterteilung: **22.03.2017**
(21) Anmeldenummer: 08802673.7
(22) Anmeldetag: 26.09.2008
(51) Int. Cl.: A61B 5/15, A61B 5/153, A61M 39/22, F16K 11/085, G01N 35/10

(54) **KÖRPERFLÜSSIGKEITSENTNAHME- UND INFUSIONSSYSTEM UND VERFAHREN ZUM BETREIBEN**
BODILY FLUID EXTRACTION AND INFUSION SYSTEM AND OPERATING METHOD
SYSTÈME D'INFUSION ET D'EXTRACTION DE FLUIDE CORPOREL ET SON PROCÉDÉ DE FONCTIONNEMENT

(30) Priorität: 27.09.2007 EP 07019012
(43) Veröffentlichungstag der Anmeldung: 09.06.2010
(73) Patentinhaber: Roche Diagnostics GmbH, 68305 Mannheim (DE); F. Hoffmann-La Roche AG, 4070 Basel (CH)
(72) Erfinder: SCHWEIGER, Gerd, A-9431 St. Stefan (AT)
(74) Vertreter: Bittner, Thomas L.
(86) Internationale Anmeldenummer: PCT/EP2008/008228
(87) Internationale Veröffentlichungsnummer: WO 2009/043555

(56) Entgegenhaltungen:
- EP-A- 0 513 789
- EP-A1- 0 376 603
- WO-A-91/18632
- WO-A-2006/039310
- FR-A- 2 535 848
- GB-A- 1 512 989
- US-A- 3 475 950
- US-A- 3 834 372
- US-A- 4 573 968
- US-A- 4 608 996
- US-A- 5 466 228

## Beschreibung

Die Erfindung bezieht sich auf ein Körperflüssigkeitsentnahme- und Infusionssystem sowie ein Verfahren zum Betreiben desselben.

### Hintergrund der Erfindung

Fluidentnahmesysteme werden beispielsweise auf dem Gebiet der Medizin-, der Labor- oder der Analysetechnik verwendet. Zum Beispiel findet eine Anwendung in Systemen statt, die der Entnahme einer Probe einer Körperflüssigkeit sowie einer anschließend wahlweise ausgeführten Analyse der entnommenen Probe in einem hierfür geeigneten Analysegerät dienen. Insbesondere werden solche Systeme dafür genutzt, einem Patienten Blut zu entnehmen und wenigstens einen Teil des entnommenen Blutes in einem Blutanalysegerät zu untersuchen. In diesem Zusammenhang kann auch vorgesehen sein, dass die Blutentnahme kontinuierlich automatisiert erfolgt. Die Entnahme kann sowohl für arterielles als auch venöses Blut erfolgen. Eine anschließende Blutanalyse erfolgt, um handlungsrelevante Informationen über den Zustand des Patienten zu gewinnen, und ermöglicht daher eine gezielte Betreuung und Therapie. Mit Hilfe von in geeigneter Weise ausgestalteten Analysegeräten können verschiedene Blutparameter untersucht werden, zu denen beispielsweise der Partialdruck von Sauerstoff und Kohlendioxid als auch die Sauerstoffsättigung gehören. Andere Parameter sind der pH-Wert, der Hämatokritwert oder der Gehalt an Natrium, Kalzium oder Kalium.

Kombiniert werden derartige Systeme zur Entnahme einer Probe der Körperflüssigkeit, beispielsweise einer Blutprobe, häufig mit Systemkomponenten für eine Infusion, wobei die Infusion gekoppelt an das Ergebnis der Analyse der entnommenen Körperflüssigkeitsprobe oder losgelöst hiervon ausführbar ist. In einem solchen System kann beispielsweise eine in Fluidleitungen integrierte Komponente wie ein 3-Wegehahn zwischen Patient und genutzten Geräten vorgesehen sein, insbesondere dem Analysegerät und einer Infusionsvorrichtung. Mit der Komponente wird Einfluss auf den Strom der Flüssigkeiten in den Fluidleitungen genommen, die Patient und angeschlossene Geräte bedarfsweise verbinden.

In Verbindung mit einem Entnahme- und Infusionssystem besteht häufig die Anforderung, dem Patienten die Infusionslösung mit variabler Flussrate über eine Leitung zuzuführen. Mittels Umkehr der Flussrichtung in der Leitung kann dem Patienten Blut entnommen werden. Eine Verteilvorrichtung in der Leitung dient dann dazu, eine Blutprobe bereitzustellen, die anschließend mit Hilfe eines Analysegerätes untersucht werden kann. Nach der Probennahme wird folgend auf eine erneute Flussrichtungsumkehr die Infusion fortgesetzt. Hierbei erfolgt gleichzeitig eine Säuberung der Fluidleitung von Blutresten.

Bei Vorrichtungen für die Entnahme einer Probe einer Körperflüssigkeit ist die Verwendung eines Septums bekannt. Hierbei wird die Probe aus der Fluidleitung unter Verwendung einer Kapillare oder einer Nadel entnommen, indem das Septum mittels der Kapillare oder der Nadel durchstochen wird, so dass die Kapillarenspitze oder die Nadelspitze in der Leitung in Kotakt mit der Körperflüssigkeit kommt. Nach der Entnahme durch die Kapillare oder die Nadel wird diese wieder aus dem Septum herausgezogen. Zur Wahrung der Sterilität muss die Kapillare oder die Nadel entweder vor jeder Entnahme desinfiziert werden, oder es wird für jede Entnahme ein neuer Einwegartikel genutzt. Hierbei treten Probleme dahingehend auf, dass die Außenseite des Septum auch zwischen zwei Probenentnahmen gegen Kontamination geschützt werden muss.

Es ist weiterhin bekannt, eine Körperflüssigkeitsprobe aus einer Fluidleitung zu entnehmen, indem die Probe durch ein Überdruckventil aus der Leitung abgegeben wird. Die so entnommene Probe kann dann dem gewünschten Analysegerät zugeführt werden. Optional kann zusätzlich ein 3-Wegehahn eingesetzt werden. Während das Überdruckventil geöffnet ist, besteht bei diesem System eine direkte Fluidverbindung zwischen der Zuleitung zu dem Analysegerät und dem Patienten. Dieses stellt ein inhärentes Sicherheitsrisiko dar. Weiterhin sind Probleme bei der Ventildichtung im Betrieb beobachtet worden, so dass es schwierig ist, hohe Anforderungen an die mit dem Überdruckventil gebildete sterile Barriere zwischen Analysegerät und Patient zu erfüllen. Aus dem Dokument US 4,608,996 ist ein 3-Wegehahn für Fluide mit drei Stellungen bekannt. In jeder der drei Stellungen ist eine Fluidverbindung zwischen genau zwei von drei äußeren Anschlüssen gebildet, nämlich einem Probenanschluss, einen Patientenanschluss und einem Ausgangsanschluss.

Aus dem Dokument US 5,758,643 sind ein Verfahren und eine Vorrichtung zum Überwachen der Blutchemie bekannt. Das System ermöglicht die Überwachung der Blutgase und Elektrolyte. Es kann Blut aus dem Kreislauf des Patienten mittels Katheter und Pumpe entnehmen. Der Aufbau der Analyseeinheit beinhaltet mehrere Sensoren, um die verschiedenen Parameter zu bestimmen. Diese Einheit befindet sich in unmittelbarer Nähe des Patientenkatheters. Nach der Analyse wird der Rest des abgenommenen Blutes wieder reinfundiert und das System mit Infusionsflüssigkeit gespült. Eine interne Überwachung ermöglicht es, den Patienten mit dem Analysator, mittels Anschlusssystemen unterschiedlicher Länge und unterschiedlichem Volumen zu verbinden.

Weiterhin ist aus dem Dokument US 5,165,406 eine Sensoranordnung für ein kombiniertes Infusions- und Blutanalysesystem bekannt. Eine Sensoranordnung weist eine Elektrodenanordnung auf, die in einem Elektrodenraum montiert ist.

Aus dem Dokument WO 2007/137285 A2 ist eine Ventilvorrichtung bekannt, bei der ein Bauteil mit einem Hohlraum in einem Gehäuse drehbar gelagert ist, so dass der Hohlraum in zwei Drehstellungen jeweils mit zwei in dem Gehäuse gebildeten Öffnungen in Fluidverbindung gebracht werden kann. Drei der am Gehäuse gebildeten Öffnungen verfügen über Anschlüsse, an die Fluidleitungen gekoppelt werden können.

In dem Dokument WO 91/18632 ist eine Ventileinrichtung beschrieben, bei der mittels eines Hohlraums in einem inneren Bauteil wahlweise zwei getrennte Fluidanschlusspaare verbunden werden können. Der Hohlraum ist gebildet, so dass ein Eingangsanschluss alternativ mit zwei Ausgängen verbunden werden kann.

Das Dokument US 3,834,372 offenbart eine Ventileinrichtung mit vier einander paarweise gegenüberliegend gebildeten Anschlüssen. In unterschiedlichen Ventilstellungen können über einen Hohlraum benachbart angeordnete oder einander gegenüberliegende Anschlüsse verbunden werden.

Auch im Dokument US 5,466,228 ist eine Ventileinrichtung beschrieben, bei der in einem Gehäuse, welches seinerseits über mehrere Anschlüsse verfügt, ein inneres Bauteil drehbar gelagert ist, in dem ein Hohlraum gebildet ist, um paarweise Anschlüsse zu verbinden. Der Hohlraum ist derart ausgeführt, dass in bestimmten Stellungen benachbart angeordnete Anschlüsse des Gehäuses miteinander verbunden werden.

Das Dokument WO 2006 / 039310 A2 beschreibt eine Vorrichtung und ein Verfahren zur automatischen Messung von Blutwerten. Die Vorrichtung ist mit einem Drei-Wege-Ventil gebildet, welches in einer ersten Stellung eine Fluidverbindung zwischen zwei Anschlüssen bereitstellt. In einer zweiten Stellung wird eine Verbindung zu einem Analysator bereitgestellt.

Das Dokument EP 0 513 789 A2 offenbart eine Vorrichtung und ein Verfahren zum Bestimmen von Komponenten von Körperflüssigkeiten mit einem Gehäuse. An dem Gehäuse sind mehrere Anschlüsse gebildet. Ein verlagerbarer Hohlraum in dem Gehäuse stellt paarweise Fluidverbindungen zwischen den Anschlüssen bereit.

In dem Dokument GB 1 512 989 sind eine Vorrichtung und ein Verfahren zur Blutgasanalyse beschrieben.

Das Dokument US 4,573,968 A offenbart ein Ventil mit einem verlagerbaren Element, welches ein Durchgang aufweist, in dem eine druckbeaufschlagte Probe eingebracht und zu einer Chromatographiesäule übertragen wird. An das Ventil wird eine Dichtflüssigkeit angeschlossen, um das druckbeaufschlagte Fluid über den Ventildurchgang auf eine Dichtoberfläche anzuwenden.

Das Dokument US 4,608,996 A offenbart ein Blutüberwachungssystem mit einem Ventil, das in drei Stellungen verlagerbar ist. Die Stellungen des Ventils ermöglichen eine Spülung des Systems, eine Überwachung des Blutdrucks und eine Entnahme einer Blutprobe.

### Zusammenfassung der Erfindung

Aufgabe der Erfindung ist es, ein Körperflüssigkeitsentnahme- und Infusionssystem mit einer Fluidverteilvorrichtung und ein Verfahren zum Betreiben des Systems zu schaffen, bei denen eine Körperflüssigkeitsprobe auch bei häufiger Nutzung zuverlässig und je nach Anwendungszweck in gewünschter Art und Weise sicher entnommen oder verteilt werden kann. Hierbei soll des Weiteren die Einhaltung hoher Sterilitätsanforderungen ermöglicht sein.

Es sind ein Körperflüssigkeitsentnahme- und Infusionssystems nach dem unabhängigen Anspruch 1 sowie ein Verfahren zum Betreiben eines Körperflüssigkeitsentnahme- und Infusionssystems nach dem unabhängigen Anspruch 11 geschaffen.

Über den patientenseitigen Anschluss gelangt eine Körperflüssigkeit des Patienten, insbesondere Blut, zu der Fluidverteilvorrichtung, nämlich in den Hohlraum. Wenn das System zur Infusion genutzt wird, dient der patientenseitige Anschluss weiterhin dazu, eine über den infusionsseitigen Anschluss zugeführte Infusionsflüssigkeit in eine Leitung zu dem Patienten einzuspeisen. Der analysatorseitige Anschluss dient zur Abgabe einer entnommenen Probe an eine Analyseeinrichtung, bedarfsweise über ein geeignetes Leitungssystem. Mit Hilfe der Analyseeinrichtung wird die Körperflüssigkeitsprobe analysiert.

Das Verfahren kann in einer bevorzugten Ausgestaltung einen Schritt umfassen, welcher als Spülschritt ausgeführt wird, zum Beispiel mittels einer physiologischen Salzlösung oder einer Infusionslösung, und bei dem der Hohlraum, einschließlich der in der gewählten Stellung ankoppelnden Anschlüsse, luftfrei gemacht werden, wodurch Luftblasen vermieden werden. Der Spülschritt kann ausgeführt werden, wenn der Hohlraum in beiden Endbereichen jeweils mit einem Anschluss verbunden ist, so dass das Spülfluid durch den Hohlraum hindurch von dem einen zu dem anderen Anschluss fließt. Aber auch ein Einströmen und Ausströmen des Spülfluids durch den gleichen Anschluss kann vorgesehen sein, wenn zum Beispiel der Hohlraum in der zum Spülen gewählten Stellung mit nur einem Anschluss in Verbindung steht.

Mit dem vorgeschlagen System ist es für den Benutzer auf einfache Art und Weise ermöglicht, die Körperflüssigkeitsprobe mittels Einströmen der Körperflüssigkeit in den Hohlraum zu sammeln und anschließend mit Hilfe einer Verlagerung des inneren Bauteils die gesammelte Körperflüssigkeitsprobe ganz oder teilweise über den zweiten Anschluss abzugeben. Ein Durchfluss zwischen dem ersten und dem ersten zugeordneten Anschluss ist mittels der bidirektionalen Fluidverbindung über den Hohlraum der Fluidverteilvorrichtung ermöglicht, der im Körperflüssigkeitsentnahme- und Infusionssystem je nach Flussrichtung zur Entnahme einer Körperflüssigkeit oder zum Infundieren einer Flüssigkeit nutzbar ist. Beim Infundieren erfolgt nicht notwendigerweise das Einbringen einer Infusionsflüssigkeit zu therapeutischen Zwecken, auch wenn die Fluidverteilvorrichtung ebenfalls in einer solchen Anwendung nutzbar ist. Vielmehr kann eine Flüssigkeit in die Fluidverteilvorrichtung eingebracht werden, um Reste der Körperflüssigkeit in den Körper zurück zu spülen.

Bei der Entnahme der Körperflüssigkeitsprobe, zum Beispiel einer Blutabnahme bei einem Patienten, kann über den ersten zugeordneten Anschluss ein Unterdruck aufgegeben werden, um für die Entnahme einen Sog zu erzeugen, der dazu führt, dass die Körperflüssigkeit in den Hohlraum einströmt. Hohe Sterilitätsanforderungen werden hierbei erfüllt, da weder in der Stellung noch in der zweiten Stellung eine Fluidverbindung zwischen dem ersten Anschluss und dem zweiten Anschluss besteht. In den Stellungen steht der Hohlraum, in welchem sich die Körperflüssigkeitsprobe sammelt, lediglich mit einem der Anschlüsse in Fluidverbindung. Ein direkter Fluss zwischen den Anschlüssen ist so wirksam unterbunden, wodurch eine effektive Barriere zwischen den Anschlüssen gebildet ist.

Das innere Bauteil mit dem Hohlraum kann in einer möglichen Ausgestaltung aus einem Vollmaterial hergestellt sein, in welches in einer einfachen Ausführung eine Bohrung für den Hohlraum eingebracht ist. Eine andere Ausführungsform sieht vor, dass das innere Bauteil als Spritzgussbauteil ausgeführt ist, so dass die Hohlraumstruktur wenigstens teilweise durch ein Werkzeug vorgegeben ist, in welches ein Kunststoffmaterial bei der Herstellung eingebracht wird.

Über den zweiten zugeordneten Anschluss kann der Hohlraum im Betrieb in einer Ausgestaltung mit Druck beaufschlagt werden, welcher insbesondere zum Ausdrücken der Körperflüssigkeitsprobe aus dem Hohlraum über den zweiten Anschluss dienen kann. Beispielsweise dient die Druckbeaufschlagung hierbei dazu, die Fluidprobe dem Analysegerät zuzuführen.

Eine bevorzugte Weiterbildung der Erfindung sieht vor, dass mit dem Hohlraum eine Kanalstruktur in dem inneren Bauteil gebildet ist. In einer einfachen Fortbildung handelt es sich bei der Kanalstruktur um einen das innere Bauteil durchsetzenden, geraden Kanal, beispielsweise in Form einer Bohrung. Die Kanalstruktur ist in nahezu beliebiger Weise ausgestaltbar und kann mit einer oder mehrerer Öffnungen gebildet sein, durch welche die Körperflüssigkeitsprobe in den Hohlraum ein- oder ausströmen kann.

Bevorzugt sieht eine Fortbildung der Erfindung vor, dass in einer dritten Stellung der Hohlraum in Fluidverbindung mit einem dritten Anschluss steht, welcher konfiguriert ist, ein Funktionsfluid in den Hohlraum zuzuführen, und getrennt ist von dem ersten Anschluss, dem ersten zugeordneten Anschluss, dem zweiten Anschluss und dem zweiten zugeordneten Anschluss. Beim Vorsehen des dritten Anschlusses ist der Hohlraum in der ersten und / oder der zweiten Stellung bevorzugt hiervon getrennt.

Eine Fortbildung der Erfindung kann vorsehen, dass das innere Bauteil mittels einer Drehbewegung zwischen den Stellungen verlagerbar ist. Ein hierfür geeignetes inneres Bauteil weist beispielsweise im wesentlichen die Form eines Zylinders auf, welcher in einem von dem äußeren Bauteil gebildeten Gehäuse angeordnet ist, zweckmäßig passgenau. In Anlehnung an die Zylinderform des inneren Bauteils kann das äußere Bauteil insgesamt einen runden Querschnitt aufweisen, wobei auf der äußeren Umlauffläche die Anschlüsse gebildet sind.

Eine Weiterbildung der Erfindung kann vorsehen, dass das innere Bauteil in die Aufnahme formschlüssig eingepasst ist. Die formschlüssige Einpassung optimiert den Schutz gegen ein nicht beabsichtigtes Überströmen des Fluids zwischen dem ersten und dem zweiten Anschluss. Hierbei ist das innere Bauteil zweckmäßig so eingepasst, dass die Verlagerung zwischen der Stellung und der weiteren Stellung nur unter Überwindung von Reibungskräften möglich ist, wodurch ein unbeabsichtigtes Verlagern zwischen den Stellungen, beispielsweise aufgrund von Vibrationen des Systems oder durch bloßes Anstoßen, vermieden ist. Auf diese Weise wird die Betriebssicherheit erhöht.

Eine vorteilhafte Ausführungsform der Erfindung sieht vor, dass die erste Stellung und die zweite Stellung benachbarte Verlagerungsstellungen des inneren Bauteils sind. Beispielsweise sind die erste Stellung und die zweite Stellung zwei benachbarte Rastposition des inneren Bauteils in dem äußeren Bauteil. Die verschiedenen Verlagerungsstellungen des inneren Bauteils sind beispielsweise dadurch charakterisiert, dass in ihnen jeweils eine Fluidverbindung zwischen dem Hohlraum und irgendeinem Anschluss besteht, der an dem äußeren Bauteil gebildet ist.

Bei einer vorteilhaften Ausgestaltung der Erfindung kann vorgesehen sein, dass der Hohlraum in Zwischenstellungen, welche von der ersten und der zweiten Stellung verschieden sind, als anschlussfreier Hohlraum gebildet ist. Bei den Zwischenstellungen handelt es sich in einer Ausführungsform um Stellungen des inneren Bauteils, die von dem inneren Bauteil relative zu dem äußeren Bauteil eingenommen werden können, in denen der Hohlraum aber nicht mit einem an dem äußeren Bauteil gebildeten Anschluss in Fluidverbindung steht. Insoweit kann in den Zwischenstellungen weder Fluid über einen Anschluss in den Hohlraum einströmen, noch diesen verlassen. In einer Ausführungsform kann dieses zweckmäßig dadurch erreicht werden, dass eine den Zugang zum Hohlraum liefernde Öffnung in den Zwischenstellungen stets von einem an dem äußeren Bauteil gebildeten Oberflächenabschnitt verschlossen ist. Zweckmäßig berühren sich in einer Weiterbildung hierbei die Öffnung umgebende Oberflächenabschnitte des inneren Bauteil mit zugeordneten Oberflächenabschnitten des äußeren Bauteils, wodurch die Abdichtung des Hohlraums weiter optimiert.

Bei einer zweckmäßigen Ausgestaltung der Erfindung kann vorgesehen sein, dass der Hohlraum zwischen der ersten und der zweiten Stellung anschlussfrei über die Zwischenstellungen verlagerbar ist. Dieses bedeutet, dass der Hohlraum während der Verlagerung zwischen den Stellungen, was über die Zwischenstellungen erfolgt, mit keinem Anschluss in Fluidverbindung tritt. Des weiteren kann vorgesehen sein, dass das Ausbilden der Fluidverbindung zwischen dem Hohlraum und einem Anschluss, mit dem der Hohlraum aufgrund der Verlagerung des inneren Bauteils neu verbunden wird, erst erfolgt, wenn die Fluidverbindung des Hohlraums zum vorangehend mit dem Hohlraum verbundenen Anschluss vollständig unterbrochen ist. Hierdurch wird insbesondere einen mögliche Kontamination des Fluids im Hohlraum vermieden. Ergänzend oder alternativ kann vorgesehen sein, dass der Hohlraum zwischen der zweiten und der dritten Stellung und / oder der ersten und der dritten Stellung anschlussfrei über die Zwischenstellungen verlagerbar ist.

Eine bevorzugte Weiterbildung der Erfindung sieht vor, dass der Hohlraum in der dritten Stellung in Fluidverbindung mit einem dritten zugeordneten Anschluss steht, welcher dem dritten Anschluss zugeordnet ist, wodurch über den Hohlraum eine bidirektionale Fluidverbindung zwischen dem dritten Anschluss und dem dritten zugeordneten Anschluss gebildet ist. Auf diese Weise ist eine durchgehende Fluidverbindung zwischen dem dritten Anschluss und dem dritten zugeordneten Anschluss geschaffen, so dass der Hohlraum beispielsweise von dem Funktionsfluid in einer Flussrichtung oder einer entgegengesetzten Flussrichtung durchströmt werden kann.

Für Ausgestaltungen des Verfahrens zum Betreiben des Körperflüssigkeitsentnahme- und Infusionssystems nach den abhängigen Unteransprüchen gelten die in Verbindung zughörigen Merkmalen vorangehend gemachten Erläuterungen entsprechend. Je nach Anwendungsfall können darüber hinausgehende Verfahrensgestaltungen bei der Nutzung der Verteilvorrichtung und der wahlweise angeschlossenen Geräte und Leitungssysteme vorgesehen sein, welche sich die konstruktiven Ausgestaltungsvarianten der und Anschlussmöglichkeiten für die Verteilvorrichtung zu Nutze machen.

### Beschreibung bevorzugter Ausführungsbeispiele der Erfindung

Die Erfindung wird im Folgenden anhand von Ausführungsbeispielen unter Bezugnahme auf Figuren einer Zeichnung näher erläutert. Hierbei zeigen:
- Fig. 1: eine schematische Darstellung eines Körperflüssigkeitsentnahme- und Infusionssystems mit einer Fluidverteilvorrichtung, wobei eine Fluidverbindung zwischen einem ersten Anschluss und einem ersten zugeordneten Anschluss gebildet ist,
- Fig. 2: eine schematische Darstellung des Körperflüssigkeitsentnahme- und Infusionssystems aus Fig. 1, wobei eine Fluidverbindung zwischen einem zweiten Anschluss und einem zweiten zugeordneten Anschluss gebildet ist,
- Fig. 3: eine schematische Darstellung des Körperflüssigkeitsentnahme- und Infusionssystems aus Fig. 2, wobei eine Körperflüssigkeitsprobe über den zweiten Anschluss abgegeben wird,
- Fig. 4: eine schematische Darstellung des Körperflüssigkeitsentnahme- und Infusionssystems aus Fig. 1, wobei zwischen einem Dritten Anschluss und einem dritten zugeordneten Anschluss eine Fluidverbindung gebildet ist,
- Fig. 5: eine schematische Darstellung des Körperflüssigkeitsentnahme- und Infusionssystems aus Fig. 4, wobei eine Infusionslösung eingebracht ist,
- Fig. 6: eine schematische Darstellung des Körperflüssigkeitsentnahme- und Infusionssystems aus Fig. 5, wobei nun eine Fluidverbindung zwischen dem ersten Anschluss und dem zugeordneten ersten Anschluss gebildet ist,
- Fig. 7: eine schematische Darstellung des Körperflüssigkeitsentnahme- und Infusionssystems aus Fig. 6, wobei eine Infusion fortgesetzt wird,
- Fig. 8: eine schematische Darstellung des Körperflüssigkeitsentnahme- und Infusionssystems nach Fig. 1, wobei eine verminderte Anzahl von Anschlüsse gebildet ist, und
- Fig. 9: eine schematische Darstellung des Körperflüssigkeitsentnahme- und Infusionssystems nach Fig. 1, wobei zusätzliche, einander zugeordnete Anschlüsse gebildet sind.

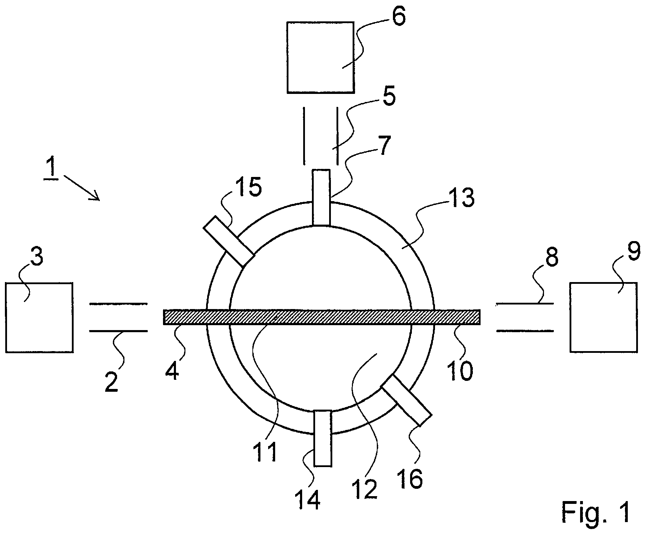

Im Folgenden wird eines Körperflüssigkeitsentnahme- und Infusionssystems mit einer Fluidverteilvorrichtung unter Bezugnahme auf die Fig. 1 bis 7 näher erläutert. Hierbei werden für gleiche Merkmale dieselben Bezugszeichen verwendet.

Fig. 1 zeigt eine schematische Darstellung einer Fluidverteilvorrichtung 1, mit welcher eine Körperflüssigkeit, insbesondere Blut, in dem System mit mehreren Fluidleitungen verteilt werden kann. Gemäß der schematischen Darstellung in Fig. 1 ist die Fluidverteilvorrichtung 1 patientenseitig über ein patientenseitiges Leitungssystem 2 mit einem Patienten 3 verbunden. Das patientenseitige Leitungssystem 2 ist mit einem ersten Anschluss 4 verbunden. Analysatorseitig ist die Fluidverteilvorrichtung 1 über ein analysatorseitiges Leitungssystem 5 mit einem Analysegerät 6 verbunden, welches eingerichtet ist, ein über die Verteilvorrichtung 1 an das Analysegerät 6 abgegebenes Fluid zu analysieren, beispielsweise eine Blutprobe. Das analysatorseitige Leitungssystem 5 ist an einen zweiten Anschluss 7 gekoppelt. Weiterhin ist die Fluidverteilvorrichtung 1 gemäß Fig. 1 infusionsseitig über ein infusionsseitiges Leitungssystem 8 an eine Infusionseinrichtung 9 gekoppelt. Auf diese Weise kann über das infusionsseitige Leitungssystem 8, welches an einen ersten zugeordneten Anschluss 10 angeschlossen ist, eine Infusionslösung durch einen Hohlraum 11, der seinerseits in einem inneren Bauteil 12 gebildet ist, zu dem patientenseitigen Leitungssystem 2 geführt werden. Bei umgekehrter Strömungsrichtung gelangt über den ersten Anschluss 4 eine Körperflüssigkeit des Patienten 3 in den Hohlraum 11, beispielsweise Blut.

Gemäß Fig. 1 sind an einem ein Gehäuse bildenden, äußeren Bauteil 13, in welchem das innere Bauteil 12 mit dem Hohlraum 11 drehbar gelagert ist, weiterhin ein zweiter zugeordneter Anschluss 14 sowie ein dritter Anschluss 15 und ein dritter zugeordneter Anschluss 16 gebildet. Einem oder mehreren der Anschlüsse, beispielsweise dem zweiten zugeordneten Anschluss 14 oder dem dritten Anschluss 15, können geeignete Ventile vorgeschaltet sein, insbesondere zur Ausbildung von getrennten Kreisläufen für unterschiedliche Funktionsfluide.

Das äußere Bauteil 13 und das innere Bauteil 12 sind beispielsweise als Spritzgussbauteile aus einem Kunststoff ausgeführt. Das innere Bauteil 12 ist passgenau in das äußere Bauteil 13 eingelassen. Mittels Drehen kann das innere Bauteil 12 unter Überwindung der Reibungskräfte zwischen zugeordneten Oberflächen von innerem und äußerem Bauteil 12, 13 in verschiedene Drehstellungen gebracht werden, in denen der Hohlraum 11 bei der in Fig. 1 dargestellten Ausführungsform mit jeweiligen Anschlüssen in Fluidverbindung steht. In Zwischenstellung (nicht dargestellt) ist der Hohlraum 11 so positioniert, dass keine Fluidverbindung zu einem der Anschlüsse besteht.

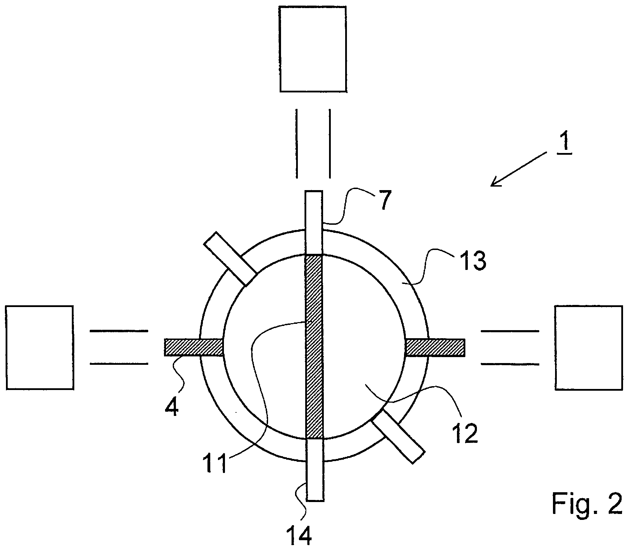

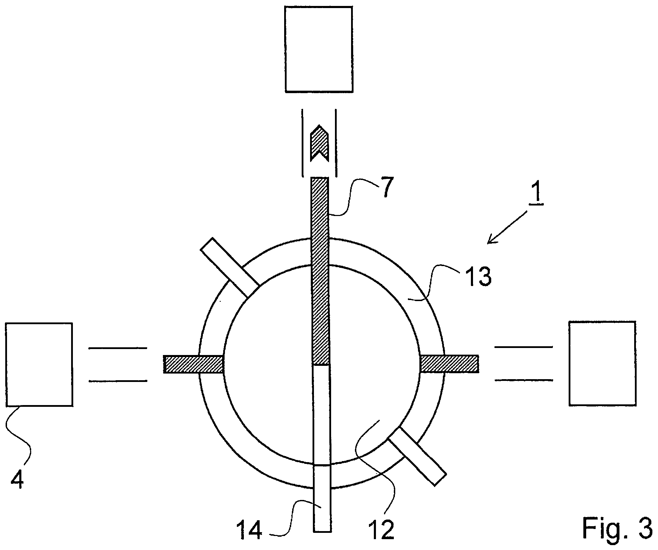

Fig. 2 zeigt eine schematische Darstellung des Körperflüssigkeitsentnahme- und Infusionssystems aus Fig. 1, wobei das innere Bauteil 12 mittels Drehen in eine Verlagerungsstellung gebracht ist, in welcher eine Fluidverbindung zwischen dem zweiten Anschluss 7 und dem zweiten zugeordneten Anschluss 14 gebildet ist. Nachdem in der in Fig. 1 gezeigten Verlagerungsstellung des inneren Bauteils 12 eine Blutprobe in den Hohlraum 11 eingeströmt ist, kann diese nun über den zweiten Anschluss 7 dem analysatorseitigen Leitungssystem 5 und schließlich dem Analysegerät 6 zugeführt werden, insbesondere mittels Druckbeaufschlagung über den zweiten zugeordneten Anschluss 14, was in Fig. 3 schematisch gezeigt ist.

Unter Ausnutzung der Fig. 2 dargestellten Stellung des Hohlraums 11 oder einer anderen Stellung, welche den Hohlraum 11 von dem ersten Anschluss 4 trennt, können Funktionsflüssigkeiten in das Analysegerät 6 eingebracht werden, zum Beispiel eine Kalibrierflüssigkeit zum Kalibrieren oder eine Kontrollflüssigkeit.

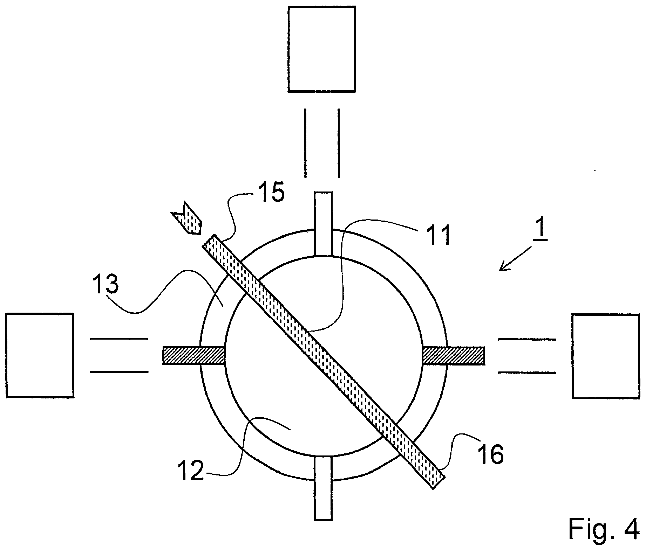

Fig. 4 zeigt eine schematische Darstellung des Körperflüssigkeitsentnahme- und Infusionssystems aus Fig. 1, wobei nun das innere Bauteil 12 in eine Verlagerungsstellung gebracht ist, in welcher über den Hohlraum 11 eine Fluidverbindung zwischen dem dritten Anschluss 15 und dem dritten zugeordneten Anschluss 16 gebildet ist. In dieser Verlagerungsstellung befindet sich das innere Bauteil 12 in einer Reinigungsposition, in welcher über den dritten Anschluss 15 ein Reinigungsfluid eingebracht werden kann. Auf diese Weise wird der Hohlraum 11 gewaschen. Weiterhin kann ein Desinfektionsmittel eingebracht werden. Ein Reinigungs- oder Waschflüssigkeit kann auch über den dritten Anschluss 15 eingebracht werden (vgl. Stellung des Hohlraums 11 in Fig. 2), wodurch eine Reinigung oder Spülung des analysatorseitigen Leitungssystems 5 realisiert werden kann.

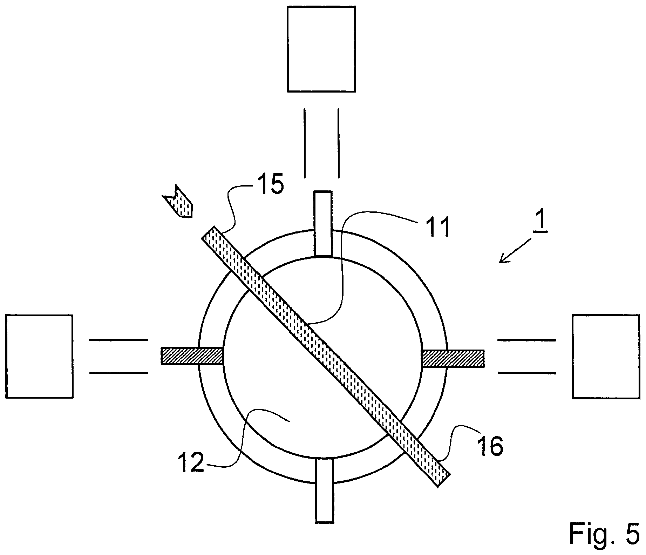

Schließlich wird, was in Fig. 5 schematisch gezeigt ist, der Hohlraum 11 mit Infusionslösung oder einer Kochsalzlösung ausgespült, um im Infusionskanal Luftblasen zu vermeiden. Hierbei kann das Einbringen der Infusionslösung auch dazu dienen, einen in dem Hohlraum 11 verbliebenen Rest der Körperflüssigkeit in den Körper des Patienten zurück zu spülen. Insoweit handelt es sich dann nicht um eine Infusionsflüssigkeit zu therapeutischen Zwecken, welche auch als Spülflüssigkeit bezeichnet werden kann.

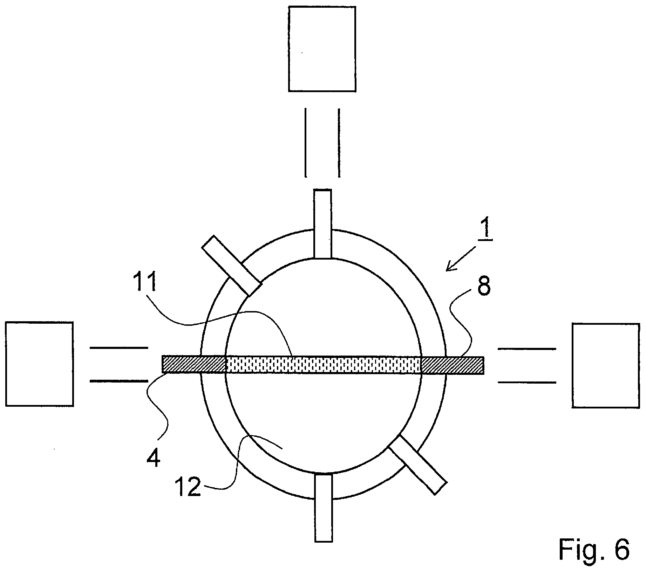

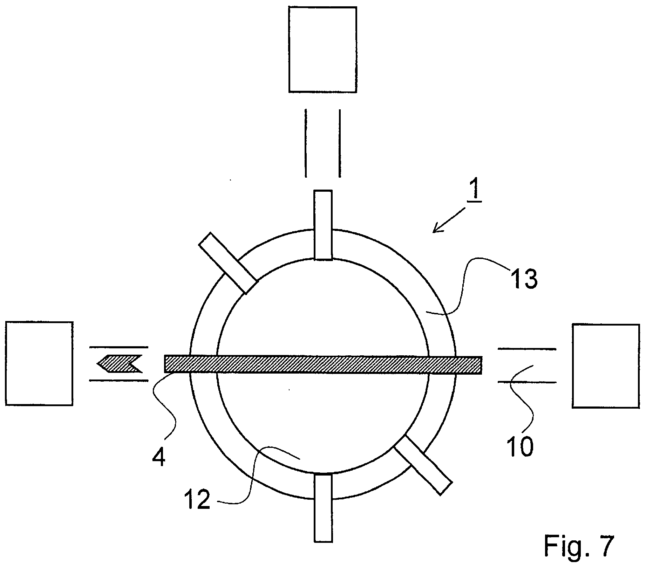

Fig. 6 zeigt dann schematisch, wie der Hohlraum 11, welcher mit der Infusionslösung oder der Kochsalzlösung gefüllt ist, nun wieder mit dem ersten Anschluss 4 und dem ersten zugeordneten Anschluss 8 in Fluidverbindung steht, um eine Infusion fortzusetzen, was dann in Fig. 7 schematisch gezeigt ist.

Die Fluidverteilvorrichtung 1 in dem Körperflüssigkeitsentnahme- und Infusionssystem aus den Fig. 1 bis 7 kann in Anlehnung an die vorangehenden Ausführungen oder wahlweise abweichend hiervon gemäß der in Tabelle 1 schematisch aufgezeigten Art und Weise betrieben werden. Hierbei sind in der Spalte "Stellung" unter Verwendung der entsprechenden Bezugszeichen die mit dem Hohlraum 11 in Verbindung stehenden Anschlüsse genannt.

**Tabelle 1**

| Schritt | Stellung | Aktion |
|---|---|---|
| 1 | 4-11-10 | Infusionsflüssigkeit fließt über eine Leitungsstrecke 8-10-11-4-2 zum Patienten |
| 2 | 4-11-10 | Blut fließt über den ersten Anschluss 4 in den Hohlraum 11 |
| 3 | | Verlagerung des inneren Bauteils 12 in die Stellung 7-11-14 |
| 4 | 7-11-14 | Blut fließt vom Hohlraum 11 über den zweiten Anschluss 7 in das Analysatorgerät 6, wonach eine Messung erfolgt. Über den zweiten zugeordneten Anschluss 14 strömt ein Funktionsfluid nach, vorzugsweise Luft. |
| 5 | | Verlagerung des inneren Bauteils 12 in die Stellung 15-11-16 |
| 6 | 15-11-16 | Der Hohlraum 11 wird mittels einer Waschflüssigkeit gereinigt. Dabei strömt die Waschflüssigkeit über den dritten Anschluss 15 ein. Im Nachgang zu dem Waschvorgang wird die Waschflüssigkeit vorzugsweise durch Luft ersetzt. |
| 7 | | Weiter mit Schritt 8 oder mit Schritt 12 |
| 8 | 15-11-16 | Im Hohlraum 11 vorhandenes Fluid wird durch eine weitere Funktionsflüssigkeit ersetzt, beispielsweise eine Kalibrier- oder eine QC-Flüssigkeit (Kontrollflüssigkeit). |
| 9 | | Verlagerung des inneren Bauteils 12 in die Stellung 7-11-14 |
| 10 | 7-11-14 | Im Hohlraum 11 befindliche weitere Funktionsflüssigkeit strömt über den zweiten Anschluss 7 und das analysatorseitige Leitungssystem 5 in das Analysatorgerät 6, wonach eine Kalibrier- oder eine QC-Messung erfolgt. |
| 11 | | Verlagerung des inneren Bauteils 12 in die Stellung 15-11-16 und weiter mit Schritt 8 oder mit Schritt 12 |
| 12 | 15-11-16 | Im Hohlraum 11 vorhandenes Fluid wird durch Infusionsflüssigkeit ersetzt. |
| 13 | | Verlagerung des inneren Bauteils 12 in die Stellung 4-11-10 |
| 14 | 4-11-10 | Infusionsflüssigkeit fließt über den ersten Anschluss 4 und das patientenseitige Leitungssystem 2 zum Patienten, wobei aus Schritt 2 in dem patientenseitigen Leitungssystem 2, dem ersten Anschluss 4, dem ersten zugeordneten Anschluss 10 und dem infusionsseitigen Leitungssystem 8 vorhandene Restflüssigkeit in den Körper zurückgespült wird. |
| 15 | 4-11-10 | weiter mit Schritt 1 |

Am dritten Anschluss 15 können ein oder mehrere Ventile angeschlossen sein, so dass wahlweise Luft oder eine von mehreren Funktionsflüssigkeiten eingebracht werden können. Optional handelt es sich bei der Waschflüssigkeit und der Infusionsflüssigkeit um ein und dieselbe Flüssigkeit. Zur Initialisierung (Inbetriebnahme) der erläuterten Anordnung mit der Fluidverteilvorrichtung 1 sind üblicherweise hier nicht näher ausgeführte, dem Schritt 1 vorgelagerte Schritte vorgesehen. Zur Abkopplung der erläuterten Anordnung gibt es hier nicht näher ausgeführte, dem der Schritte 1 bis 15 nachgelagerte Schritte.

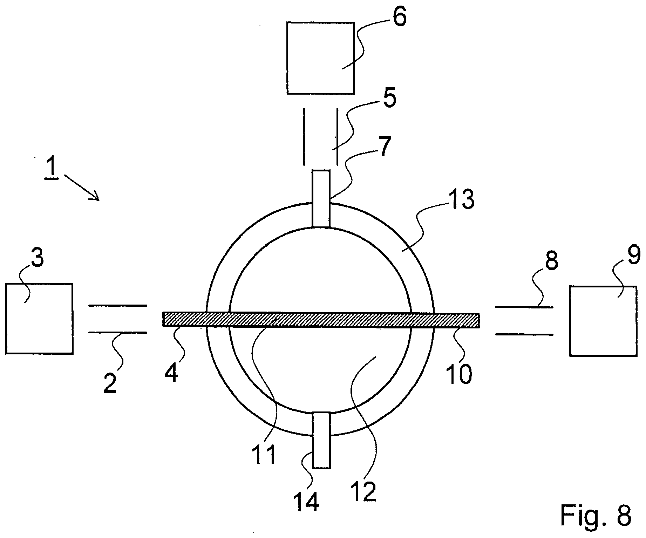

Fig. 8 zeigt eine schematische Darstellung einer Fluidverteilvorrichtung 1, wobei eine verminderte Anzahl von Anschlüsse gebildet ist. Die Fluidverteilvorrichtung 1 aus Fig. 8 kann gemäß der in Tabelle 2 schematisch aufgezeigten Art und Weise betrieben werden. Hierbei sind wieder in der Spalte "Stellung" unter Verwendung der entsprechenden Bezugszeichen die mit dem Hohlraum 11 in Verbindung stehenden Anschlüsse genannt.

**Tabelle 2**

| Schritt | Stellung | Aktion |
|---|---|---|
| 1 | 4-11-10 | Infusionsflüssigkeit fließt über eine Leitungsstrecke 8-10-11-4-2 zum Patienten |
| 2 | 4-11-10 | Blut fließt über den ersten Anschluss 4 in den Hohlraum 11 |
| 3 | | Verlagerung des inneren Bauteils 12 in die Stellung 7-11-14 |
| 4 | 7-11-14 | Blut fließt vom Hohlraum 11 über den zweiten Anschluss 7 in das Analysegerät 6, wonach eine Messung erfolgt. Über den zweiten zugeordneten Anschluss 14 strömt ein Funktionsfluid nach, vorzugsweise Luft. |
| 5 | 7-11-14 | Eine Leitungsstrecke 14-11-7-5 wird mit einer Waschflüssigkeit gereinigt. Hierbei strömt die Waschflüssigkeit wird über den zweiten zugeordneten Anschluss 14 oder über das analysatorseitige Leitungssystem 5 ein. Im Nachgang zu dem Waschvorgang wird die Waschflüssigkeit ersetzt, vorzugsweise durch Luft. |
| 6 (optional) | 7-11-14 | Eine oder mehrere weitere Funktionsflüssigkeiten werden sequenziell über den zweiten zugeordneten Anschluss 14 eingebracht, gegebenenfalls durch Luftpakete getrennt, und strömen über eine Leitungsstrecke 14-11-7 das Analysegerät 6, wonach Kalibrier- bzw. QC-Messungen erfolgen. |
| 7 | 7-11-14 | Aus den Schritten 5 oder 6 vorhandene Funktionsflüssigkeit oder Luft wird durch Infusionsflüssigkeit ersetzt. |
| 8 | | Verlagerung des inneren Bauteils 12 in die Stellung 4-11-10 |
| 9 | 4-11-10 | Infusionsflüssigkeit fließt zum Patienten, wobei aus Schritt 2 in dem patientenseitigen Leitungssystem 2, dem ersten Anschluss 4, dem ersten zugeordneten Anschluss 10 und dem infusionsseitigen Leitungssystem 8 vorhandene Restflüssigkeit in den Körper zurückgespült wird. |
| 10 | 4-11-10 | weiter mit Schritt 1 |

Am zweiten zugeordneten Anschluss 14 können ein oder mehrere Ventile angeschlossen sein, so dass wahlweise Luft oder eine von mehreren Funktionsflüssigkeiten eingebracht werden können. Optional handelt es sich bei der Waschflüssigkeit und Infusionsflüssigkeit um ein und dieselbe Flüssigkeit. Zur Initialisierung (Inbetriebnahme) der erläuterten Anordnung mit der Fluidverteilvorrichtung 1 gibt es hier nicht näher ausgeführte, dem Schritt 1 vorgelagerte Schritte. Zur Abkopplung der erläuterten Anordnung können hier nicht näher ausgeführte, einem der Schritte 1 bis 10 nachgelagerte Schritte vorgesehen sein.

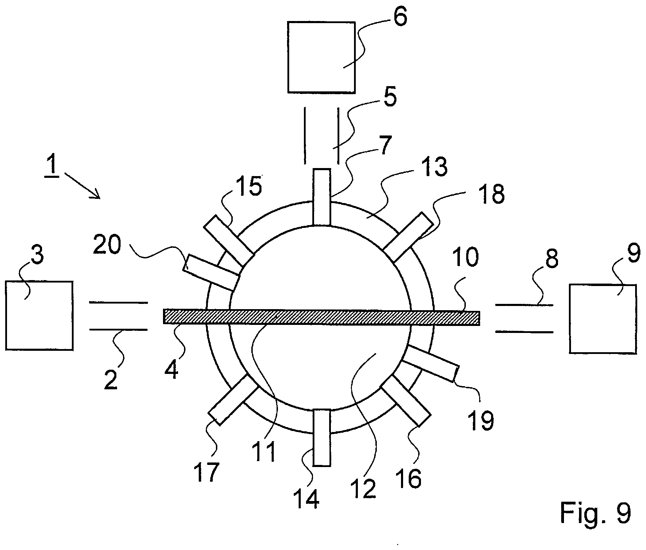

Fig. 9 zeigt eine schematische Darstellung des Körperflüssigkeitsentnahme- und Infusionssystems nach Fig. 1, wobei zusätzliche, einander zugeordnete Anschlüsse 17, 18 und 19, 20 gebildet sind. Unter Verwendung der zusätzlichen, einander zugeordneten Anschlüsse 17, 18 und 19, 20 können weitere separate Kreisläufe realisiert werden.

Die vorangehend beschriebenen Betriebszyklen der Verteilvorrichtung in ihren verschiedenen Ausgestaltungen bilden bevorzugte Ausgestaltungen, die jedoch anderen Anforderungen im konkreten Anwendungsfall geändert und angepasst werden können.

## Patentansprüche

1. Körperflüssigkeitsentnahme- und Infusionssystem, insbesondere Blutentnahme- und Infusionssystem, mit
- einem patientenseitigen Leitungssystem (2),
- einem analysatorseitigen Leitungssystem (5),
- einem infusionsseitigen Leitungssystem (8),
- einem Analysegerät (6),
- einer Infusionseinrichtung (9),
- Mitteln zur Druckbeaufschlagung und
- einer Fluidverteilvorrichtung (1) mit einem äußeren Bauteil (13), an welchem mehrere Anschlüsse gebildet sind, und einem inneren Bauteil (12), welches wenigstens teilweise in einer Aufnahme in dem äußeren Bauteil (13) angeordnet ist, zwischen Stellungen verlagerbar ist und einen für eine Fluidaufnahme und -abgabe konfigurierten Hohlraum (11) aufweist, wobei:
- an der Fluidverteilvorrichtung (1)
- ein erster Anschluss (4) als ein patientenseitiger Anschluss gebildet ist, welcher mit dem patientenseitigen Leitungssystem (2) verbunden ist,
- ein erster zugeordneter Anschluss (10), welcher dem ersten Anschluss (4) zugeordnet und über das infusionsseitige Leitungssystem (8) mit der Infusionseinrichtung (9) verbunden ist, als ein infusionsseitiger Anschluss gebildet ist,
- ein zweiter Anschluss (7), welcher über das analysatorseitige Leitungssystem (5) mit dem Analysegerät (6) verbunden ist, als ein analysatorseitiger Anschluss gebildet ist, und
- ein zweiter zugeordneter Anschluss (14) gebildet ist, welcher dem zweiten Anschluss (7) zugeordnet und mit den Mitteln zur Druckbeaufschlagung verbunden ist,
- in einer ersten Stellung des inneren Bauteils (12) über den Hohlraum (11) zwischen dem ersten Anschluss (4) und dem ersten zugeordneten Anschluss (10) eine erste bidirektionale Fluidverbindung gebildet ist und der Hohlraum (11) getrennt ist von dem zweiten Anschluss (7) und dem zweiten zugeordneten Anschluss (14), und
- in einer zweiten Stellung über den Hohlraum (11) zwischen dem zweiten Anschluss (7) und dem zweiten zugeordneten Anschluss (14) eine zweite bidirektionale Fluidverbindung gebildet ist und der Hohlraum (11) getrennt ist von dem ersten Anschluss (4) und dem ersten zugeordneten Anschluss (10).

2. System nach Anspruch 1, **dadurch gekennzeichnet, dass** mit dem Hohlraum (11) eine Kanalstruktur in dem inneren Bauteil (12) gebildet ist.

3. System nach Anspruch 1 oder 2, **dadurch gekennzeichnet, dass** in einer dritten Stellung der Hohlraum (11) in Fluidverbindung mit einem dritten Anschluss (15) steht, welcher konfiguriert ist, ein Funktionsfluid in den Hohlraum (11) zuzuführen, und getrennt ist von dem ersten Anschluss (4), dem ersten zugeordneten Anschluss (10), dem zweiten Anschluss (7) und dem zweiten zugeordneten Anschluss (14).

4. System nach Anspruch 3, **dadurch gekennzeichnet, dass** in der ersten Stellung der Hohlraum (11) getrennt ist von dem dritten Anschluss (15).

5. System nach Anspruch 3 oder 4, **dadurch gekennzeichnet, dass** in der zweiten Stellung der Hohlraum (11) getrennt ist von dem dritten Anschluss (15).

6. System nach mindestens einem der vorangehenden Ansprüche, **dadurch gekennzeichnet, dass** das innere Bauteil (12) mittels einer Drehbewegung zwischen den Stellungen verlagerbar ist.

7. System nach mindestens einem der vorangehenden Ansprüche, **dadurch gekenn-zeichnet,** dass das innere Bauteil (12) in die Aufnahme formschlüssig eingepasst ist.

8. System nach mindestens einem der vorangehenden Ansprüche, **dadurch gekennzeichnet, dass** die erste Stellung und die zweite Stellung benachbarte Verlagerungsstellungen des inneren Bauteils (12) sind.

9. System nach mindestens einem der vorangehenden Ansprüche, **dadurch gekennzeichnet, dass** der Hohlraum (11) in Zwischenstellungen, welche von der ersten und der zweiten Stellung verschieden sind, als anschlussfreier Hohlraum gebildet ist.

10. System nach Anspruch 9, **dadurch gekennzeichnet, dass** der Hohlraum (11) zwischen der ersten und der zweiten Stellung anschlussfrei über die Zwischenstellungen verlagerbar ist.

11. Verfahren zum Betreiben eines Körperflüssigkeitsentnahme- und Infusionssystems gemäß einem der vorangehenden Ansprüche 1 bis 10, insbesondere eines Blutentnahme- und Infusionssystems, mit einer Fluidverteilvorrichtung (1) mit einem äußeren Bauteil (13), an welchem mehrere Anschlüsse gebildet sind, und einem inneren Bauteil (12), welches wenigstens teilweise in einer Aufnahme in dem äußeren Bauteil (13) angeordnet ist, zwischen Stellungen verlagerbar ist und einen für eine Fluidaufnahme und -abgabe konfigurierten Hohlraum (11) aufweist, wobei an der Fluidverteilvorrichtung (1) ein erster Anschluss (4) als ein patientenseitiger Anschluss, ein erster zugeordneter Anschluss (10), welcher dem ersten Anschluss (4) zugeordnet ist, als ein infusionsseitiger Anschluss und ein zweiter Anschluss (7) als ein analysatorseitiger Anschluss gebildet sind und wobei das Verfahren die folgenden Schritte aufweist:
- Anordnen des inneren Bauteils (12) in einer ersten Stellung, in welcher über den Hohlraum (11) zwischen dem ersten Anschluss (4) und dem ersten zugeordneten Anschluss (10) eine bidirektionale Fluidverbindung gebildet und der Hohlraum (11) getrennt wird von dem zweiten Anschluss (7) und einem zweiten zugeordneten Anschluss (14), welcher dem zweiten Anschluss (7) zugeordnet ist,
- Einbringen einer Körperflüssigkeitsprobe über den ersten Anschluss in den Hohlraum (11),
- Verlagern des inneren Bauteils (12) in eine zweite Stellung, in welcher der Hohlraum (11) in Fluidverbindung mit dem zweiten Anschluss (7) steht, wobei der Hohlraum (11) beim Anordnen des inneren Bauteils (12) in der zweiten Stellung getrennt wird von dem ersten Anschluss (4) und dem ersten zugeordneten Anschluss (10),
- wenigstens teilweises Abgeben der Körperflüssigkeitsprobe aus dem inneren Hohlraum (11) über den zweiten Anschluss (7) an eine Analyseeinrichtung (6),
- Verlagern des inneren Bauteils (12) in die erste Stellung und
- Bereitstellen einer Infusionsflüssigkeit mittels einer Infusionseinrichtung (9) an dem ersten zugeordneten Anschluss (10) für eine Abgabe über den Hohlraum (11) und den ersten Anschluss (4).

12. Verfahren nach Anspruch 11, **dadurch gekennzeichnet, dass** das innere Bauteil (12) in eine dritte Stellung verlagert wird, in welcher der Hohlraum (11) in Fluidverbindung mit einem dritten Anschluss (15) steht, wobei der Hohlraum (11) beim Anordnen des inneren Bauteils (12) in der dritten Stellung getrennt wird von dem ersten Anschluss (4), dem ersten zugeordneten Anschluss (10), dem zweiten Anschluss (7) und dem zweiten zugeordneten Anschluss (14).

13. Verfahren nach einem der Ansprüche 11 und 12, **dadurch gekennzeichnet, dass** beim Einbringen der Körperflüssigkeitsprobe über den ersten Anschluss in den Hohlraum (11) eine entnommene Blutprobe eingebracht wird.

14. Verfahren nach mindestens einem der Ansprüche 11 bis 13, **dadurch gekennzeich**n e t, dass der Hohlraum (11) mit einem Funktionsfluid gespült wird, indem dem Hohlraum (11) das Funktionsfluid zugeführt wird.

## Claims

1. A system for the extraction and infusion of body fluids, in particular the extraction and infusion of blood, having
- a patient-side fluid distribution system (2),
- an analyzer-side fluid distribution system (5),
- an infusion-side fluid distribution system (8),
- an analyzing apparatus (6),
- an infusion apparatus (9),
- means for pressurizing, and
- a fluid distribution device (1) having an outer structural element (13) at which several connection ports are formed and an inner structural element (12) which, at least partially, is arranged within a seat in the outer structural element (13), is displaceable between positions and has a hollow space (11) configured for an intake and discharge of a fluid, wherein:
- at the fluid distribution device (1)
- a first connection port (4) is formed as a patient-side connection port, which is connected to the patient-side fluid distribution system (2)
- a first assigned connection port (10) is formed as an infusion-side connection port, which is assigned to the first connection port (4) and connected to the infusion apparatus (9) through the infusion-side fluid distribution system (8),
- a second connection port (7) is formed as an analyzer-side connection port, which is connected to the analyzing apparatus (6) through the analyzer-side fluid distribution system (5), and
- a second assigned connection port (14) is formed, which is assigned to the second connection port (7) and connected to the means for pressurizing,
- in a first position of the inner structural element (12) a first bi-directional fluid connection through the hollow space (11) is formed between the first connection port (4) and the first assigned connection port with the hollow space (11) being separated from the second connection port (7) and the second assigned connection port (14), and
- in a second position a second bi-directional fluid connection is formed through the hollow space (11) between the second connection port (7) and the second assigned connection port (14) with the hollow space (11) being separated from the first connection port (4) and the first assigned connection port (10).

2. The system in accordance with Claim **1, characterized in that** a channel structure in the inner structural element (12) is formed with the hollow space (11).

3. The system in accordance with Claim 1 or **2, characterized in that** in a third position the hollow space (11) is in fluid connection with a third connection port (15) which is configured to feed a function fluid into the hollow space (11) and is separated from the first connection port (4), the first assigned connection port (10), the second connection port (7) and the second assigned connection port (14).

4. The system in accordance with Claim 3, **characterized in that** in the first position the hollow space (11) is separated from the third connection port (15).

5. The system in accordance with Claim 3 or 4, **characterized in that** in the second position the hollow space (11) is separated from the third connection port (15).

6. The system in accordance with at least one of the preceding claims, **characterized in that** the inner structural element (12) is displaceable between the positions by a rotary movement.

7. The system in accordance with at least one of the preceding claims, **characterized in that** the inner structural element (12) and the seat have a positive fit.

8. The system in accordance with at least one of the preceding claims, **characterized in that** the first position and the second position are adjacent displacement positions of the inner structural element (12).

9. The system in accordance with at least one of the preceding claims, **characterized in that** the hollow space (11) in intermediate locations which differ from the first and second position is formed as connection-free hollow space.

10. The system in accordance with Claim 9, **characterized in that** the hollow space (11) is displaceable with no connection via the intermediate locations between the first and second position.

11. A method for operating a system for the extraction and infusion of a body fluid in accordance with one of the preceding claims 1 to 10, in particular a system for the extraction and infusion of blood, having a fluid distribution device (1) having an outer structural element (13) at which several connection ports are formed and an inner structural element (12) which, at least partially, is arranged within a seat in the outer structural element (13), is displaceable between positions and has a hollow space (11) configured for the intake and discharge of a fluid, wherein at the fluid distribution device (1) a first connection port (4) as a patient-side connection port, a first assigned connection port (10) which is assigned to the first connection port (4) as an infusion-side connection port, and a second connection port (7) as an analyzer-side connection port are formed, the method comprising the following steps:
- arranging the inner structural element (12) in a first position in which a bi-directional fluid connection through the hollow space (11) is formed between the first connection port (4) and the first assigned connection port (10), and the hollow space (11) is separated from the second connection port (7) and a second assigned connection port (14) which is assigned to the second connection port (7),
- introducing a body fluid sample through the first connection port into the hollow space (11),
- displacing the inner structural element (12) in a second position in which the hollow space (11) is in fluid connection with the second connection port (7), wherein the hollow space (11) is separated from the first connection port (4) and the first assigned connection port (10) by the arrangement of the inner structural element (12) in the second position,
- at least partially discharging of the body fluid sample from the inner hollow space (11) to an analyzing apparatus (6) through the second connection port (7),
- displacing the inner structural element (12) to the first position, and
- providing an infusion fluid by means of an infusion apparatus (9) to the first assigned connection port (10) for discharge through the hollow space (11) and the first connection port (4).

12. The method in accordance with Claim 11, **characterized in that** the inner structural element (12) is displaced in a third position in which the hollow space (11) is in fluid connection with a third connection port (15), wherein the hollow space (11) is separated from the fist connection port (4), the first assigned connection part (10), the second connection port (7) and the second assigned connection port (14) by the arrangement of the inner structural element (12) in the third position.

13. The method in accordance with one of the Claims 11 and 12, **characterized in that** by introducing the body fluid sample through the first connection port into the hollow space (11) a taken blood sample is introduced.

14. The method in accordance with at least one of the Claims 11 to 13, **characterized in that** the hollow space (11) is rinsed with a function fluid with the function fluid being fed to the hollow space (11).

## Revendications

1. Système de prélèvement et de perfusion de liquide physiologique, en particulier système de prélèvement et de perfusion de sang, comportant
- un système de canalisations au niveau du patient (2),
- un système de canalisations au niveau de l'analyseur (5),
- un système de canalisations au niveau de la perfusion (8),
- un appareil d'analyse (6),
- un dispositif de perfusion (9),
- des moyens d'application de pression et
- un dispositif répartiteur de fluide (1), comportant une composante extérieure (13) sur laquelle sont constitués plusieurs raccordements et une composante intérieure (12) qui est disposée au moins partiellement dans un support de la composante extérieure (13), qui peut être permuté entre des positions et présente une cavité (11) configurée pour recevoir et délivrer du fluide, dans lequel:
- au niveau du dispositif répartiteur de fluide (1),
- est constitué un premier raccordement (4) sous forme d'un raccordement au niveau du patient qui est connecté au système de canalisations au niveau du patient (2),
- est constitué un premier raccordement associé (10), qui est associé au premier raccordement (4) et est connecté par le système de canalisations au niveau de la perfusion (8) au dispositif de perfusion (9) sous forme d'un raccordement au niveau de la perfusion,
- est constitué un deuxième raccordement (7) qui est connecté par le système de canalisations au niveau de l'analyseur (5) à l'appareil d'analyse (6) et réalisé sous forme d'un raccordement au niveau de la perfusion,
- est constitué un deuxième raccordement associé (14) qui est associé au deuxième raccordement (7) et est connecté aux moyens d'application de pression,
- dans une première position de la composante intérieure (12) au-dessus de la cavité (11) entre le premier raccordement (4) et le premier raccordement associé (10), une première connexion bidirectionnelle de fluide est constituée et la cavité (11) est séparée du deuxième raccordement (7) et du deuxième raccordement associé (14), et
- dans une deuxième position au-dessus de la cavité (11) entre le deuxième raccordement (7) et le deuxième raccordement associé (14), une deuxième connexion bidirectionnelle de fluide est constituée et la cavité (11) est séparée du premier raccordement (4) et du premier raccordement associé (10).

2. Système selon la revendication 1, **caractérisé en ce qu'**une structure de canal est formée dans la composante intérieure (12) par la cavité (11).

3. Système selon la revendication 1 ou 2, **caractérisé en ce que**, dans une troisième position, la cavité (11) est en connexion de fluide avec un troisième raccordement (15) qui est configuré pour acheminer un fluide fonctionnel dans la cavité (11) et est séparé du premier raccordement (4), du premier raccordement associé (10), du deuxième raccordement (7) et du deuxième raccordement associé (14).

4. Système selon la revendication 3, **caractérisé en ce que**, dans la première position, la cavité (11) est séparée du troisième raccordement (15).

5. Système selon la revendication 3 ou 4, **caractérisé en ce que**, dans la deuxième position, la cavité (11) est séparée du troisième raccordement (15).

6. Système selon au moins une des revendications précédentes, **caractérisé en ce que** la composante intérieure (12) peut être permutée entre les positions au moyen d'un mouvement rotatif.

7. Système selon au moins une des revendications précédentes, **caractérisé en ce que** la composante intérieure (12) est ajustée en correspondance géométrique dans le support.

8. Système selon au moins une des revendications précédentes, **caractérisé en ce que** la première position et la deuxième position sont des positions de permutation voisines de la composante intérieure (12).

9. Système selon au moins une des revendications précédentes, **caractérisé en ce que** la cavité (11) est réalisée sous forme d'une cavité sans raccordement dans des positions intermédiaires qui sont différentes de la première et de la deuxième position.

10. Système selon la revendication 9, **caractérisé en ce que** la cavité (11) peut être permutée de la première à la deuxième position sans raccordement en passant par les positions intermédiaires.

11. Procédé d'utilisation d'un système de prélèvement et de perfusion de liquide physiologique selon une des revendications précédentes 1 à 10, en particulier système de prélèvement et de perfusion de sang, comportant un dispositif répartiteur de fluide (1) doté d'une composante extérieure (13) sur laquelle sont constitués plusieurs raccordements et d'une composante intérieure (12) qui est disposée au moins partiellement dans un support de la composante extérieure (13), pouvant être permuté entre des positions et présentant une cavité (11) configurée pour un prélèvement et une délivrance de fluide, étant constitué au niveau de dispositif répartiteur de fluide (1) un premier raccordement (4) sous forme d'un raccordement au niveau du patient, un premier raccordement associé (10) qui est associé au premier raccordement (4) sous forme d'un raccordement au niveau de la perfusion et un deuxième raccordement (7) sous forme d'un raccordement au niveau de l'analyseur et le procédé comprenant les étapes suivantes :
- disposition de la composante intérieure (12) dans une première position dans laquelle est constituée au-dessus de la cavité (11), entre le premier raccordement (4) et le premier raccordement associé (10), une connexion bidirectionnelle de fluide et la cavité (11) étant séparée du deuxième raccordement (7) et d'un deuxième raccordement associé (14) qui est associé au deuxième raccordement (7),
- introduction d'un échantillon de liquide physiologique par l'intermédiaire du premier raccordement dans la cavité (11),
- permutation de la composante intérieure (12) dans une deuxième position dans laquelle la cavité (11) est en connexion de fluide avec le deuxième raccordement (7), la cavité (11) étant, en cas de disposition de la composante intérieure (12) dans la deuxième position, séparée du premier raccordement (4) et du premier raccordement associé (10),
- délivrance au moins partielle de l'échantillon de liquide physiologique depuis la cavité interne (11) par le deuxième raccordement (7) à un dispositif d'analyse (6),
- permutation de la composante intérieure (12) dans la première position et
- mise à disposition d'un liquide de perfusion au moyen d'un dispositif de perfusion (9) au niveau du premier raccordement associé (10) pour une délivrance par l'intermédiaire de la cavité (11) et du premier raccordement (4).

12. Procédé selon la revendication 11, **caractérisé en ce que** la composante intérieure (12) est permutée dans une troisième position dans laquelle la cavité (11) est en connexion de fluide avec un troisième raccordement (15), la cavité (11) étant, en cas de disposition de la composante intérieure (12) dans la troisième position, séparée du premier raccordement (4), du premier raccordement associé (10), du deuxième raccordement (7) et du deuxième raccordement associé (14).

13. Procédé selon une des revendications 11 et 12, **caractérisé en ce que**, à l'introduction de l'échantillon de liquide physiologique par le premier raccordement dans la cavité (11), un échantillon de sang prélevé est incorporé.

14. Procédé selon au moins une des revendications 11 à 13, **caractérisé en ce que** la cavité (11) est rincée avec un fluide fonctionnel en acheminant le fluide fonctionnel vers la cavité (11).
